# EUROPÄISCHE PATENTANMELDUNG

(11) **EP 1 800 713 A1**
(43) Veröffentlichungstag der Anmeldung: **27.06.2007**
(21) Anmeldenummer: 05028266.4
(22) Anmeldetag: 22.12.2005
(51) Int. Cl.: A61P 31/00, A23K 1/16, A61K 31/765

(54) **Verwendung von Alkoxylaten ein- und mehrwertiger Alkohole oder eines Derivats davon als Antibiotikaersatz in der Tierenährung**

(71) Anmelder: BASF Aktiengesellschaft, 67056 Ludwigshafen (DE)
(72) Erfinder: Klumpe, Markus, 68163 Mannheim (DE); Grundler, Otto Johann, 67069 Ludwigshafen (DE); Langbein, Inge, 67227 Frankenthal (DE); Seelmann-Eggebert, Hans-Peter, 67117 Limburgerhof (DE)
(74) Vertreter: Huhn, Michael

(57) **Zusammenfassung**

Die vorliegende Erfindung bezieht sich auf die Verwendung von Alkoxylaten ein- und mehrwertiger Alkohole oder einem Derivat davon als Antibiotikumersatz bei der Aufzucht und Haltung von Vieh. Dadurch lässt sich eine erhöhte Gewichtszunahme und eine bessere Gesundheit der Tiere erreichen.

## Beschreibung

Die vorliegende Erfindung bezieht sich auf eine neuartige Verwendung von Alkoxylaten ein- und mehrwertiger Alkohole oder eines Derivats davon in der Tierernährung, und zwar als Antibiotikaersatz. Hierbei werden die Alkoxylate ein- und mehrwertiger Alkohole oder ihre Derivate vorzugsweise dem Futtermittel zugesetzt.

Es ist seit vielen Jahren bekannt, Tierfutter Antibiotika beizumischen. Dadurch werden die Tiergesundheit und die Stallhygiene verbessert und so ein Schutz vor Infektionen erreicht. Es resultiert generell ein verbessertes Wachstum (schnellere Gewichtszunahme) der Tiere

(Leistungsförderung).

Das Erfüllen der vorstehend genannten Punkte ist sowohl ein ethisches als auch ein wirtschaftliches Anliegen, da die Tiere ein bedeutender Teil des Unternehmenskapitals eines Landwirts sind. Der aus dem Wettbewerb resultierende Zwang zur Kostensenkung hat über Jahre zu Systemen der Tierhaltung geführt, die in Anbetracht einer gewissen notwendigen, artgerechten Tierhaltung zunehmend kritisch hinterfragt werden.

Eine die Leistung der Tiere fördernde Fütterung liegt schon aus wirtschaftlichen Gründen im Interesse des Tierhalters. Das Futtermittelrecht knüpft hier an. Zweck des Futtermittelgesetzes ist es unter anderem, die tierische Erzeugung im Hinblick auf die Leistungsfähigkeit der Nutztiere und die Qualität zu fördern sowie sicherzustellen, dass die Gesundheit der Tiere durch Futtermittel nicht beeinträchtigt wird. Die Qualität der durch tierische Erzeugung genannten Produkte ist auch wichtig im Hinblick auf die Unbedenklichkeit für die Gesundheit des Menschen.

Das Auftreten von Tierkrankheiten und Tierseuchen ist häufig mit der Tötung von Tieren und großen wirtschaftlichen Verlusten verbunden. Aus Sicht der Tierhygiene gilt es, Tiere und Tierbestände vor Infektionskrankheiten, insbesondere Tierseuchen, zu bewahren. Dazu müssen der Eintrag von Krankheitserregern in den Bestand, ihre Ausbreitung im Bestand und die Weiterverbreitung in andere Bestände verhindert werden. Entsprechende hygienische Anforderungen werden beispielsweise durch die Schweinehaltungshygiene-Verordnung und die Hühner-Salmonellen-Verordnung festgelegt.

Verbunden ist mit diesem Erfordernis jedoch auch die Frage nach dem Medikamenteneinsatz, insbesondere zur Prophylaxe und zur Leistungsförderung. Prophylaktischer Medikamenteneinsatz der auch der Leistungsförderung dient, ist sowohl aus Sicht des Tierschutzes wie auch aus der Perspektive des vorsorgenden gesundheitlichen Verbraucherschutzes und der Lebensmittelsicherheit kritisch zu sehen.

Bei der Haltung von Geflügel und weiteren Schlachttieren wie Schweinen und Rindern ist es inzwischen zur Routine geworden, so genannte antimikrobielle Wachstumsförderer (AMWF) als Futtermittelzusatz einzusetzen. Dadurch wird - wie der Name bereits sagt - das Wachstum der Tiere gefördert.

Für den Einsatz als AMWF hatte die EU vor langem entschieden, keine Antibiotika zuzulassen, die in der Humanmedizin verwendet werden. Allerdings gab es Ausnahmen wie das in der Geflügelzucht häufig gebrauchte Bacitracin, das in der Humanmedizin zur Behandlung oberflächlicher Infektionen von Auge, Ohr und Darm verwendet wird. In den letzten Jahren hat sich jedoch herausgestellt, dass einige der zugelassenen AMWF Kreuzresistenz gegen Antibiotika der Humanmedizin bedingen, weil sie in ihrer chemischen Struktur mit medizinisch wichtigen Antibiotika so nahe verwandt sind, dass eine Selektion von Keimen erfolgt, die den gleichen Resistenzmechanismus haben. Ab 1999 wurden daher in der EU aus diesen Gründen die Zulassungen für eine Reihe von Antibiotika als AMWF ausgesetzt, darunter auch von Bacitracin.

Die gebildeten resistenten Stämme können auf unterschiedliche Weise zum Menschen gelangen und teilweise fatale Folgen auslösen. Beispielsweise können antibiotikumresistente Keime auf Fleisch einerseits ein direktes erhöhtes Gesundheitsrisiko darstellen, andererseits können sie dieses Risiko auch indirekt erhöhen, indem die Resistenzen an im menschlichen Körper vorhandene Keime weitergegeben werden. Dieses Resistenzreservoir kann später einen medizinisch notwendigen Einsatz von Antibiotika zur Bekämpfung einer Infektionskrankheit erschweren oder gar unmöglich machen.

Aus den Gefahren des übermäßigen Einsatzes von Antibiotika hat der Gesetzgeber gelernt, und es wurden entsprechende Konsequenzen gezogen. So ist in der EU ein Verbot von bestimmten AMWF, nämlich von Fütterungsantibiotika, bald zu erwarten.

Aus der Sicht der Tierzüchter ist jedoch ein solches Verbot ein wirtschaftlicher Nachteil, da bis zur Schlachtreife der Tiere deutlich mehr Zeit verstreicht. Weiterhin können die Tiere in vielen Fällen anfälliger für Infektionen werden. Es wird deswegen nach Alternativen zu Fütterungsantibiotika gesucht.

Eine Alternative liegt dabei in der Verabreichung von Kaliumdiformiat, das aufgrund seiner kristallinen Struktur einfach zu handhaben ist, verglichen der freien Säure (Ameisensäure). Ameisensäure und Kaliumdiformiate bieten einen antimikrobiellen Effekt und können die bisher verwendeten Antibiotika unter Wachstumsförderer AMWF durchaus ersetzen. Ein solches Produkt ist von der Anmelderin unter dem Namen Formi® kommerziell erhältlich.

Polyethylenglykole sind physiologisch inerte, mittel- bis hochmolekulare Flüssigkeiten oder Pulver. Sie sind generell wasserlöslich und werden in den unterschiedlichsten Anwendungen eingesetzt.

So ist es zum Beispiel aus der WO 96/11585 bekannt, Tierfutter zur besseren Verwertung mit Polyethylenglykol und bestimmten Derivaten davon zu versetzen. Der Effekt des Polyethylenglykols wird dabei in der erhöhten Verfügbarkeit der in Pulver- oder Granulatform vorliegenden Futtersubstanzen erklärt.

WO 03/061672 offenbart die Verwendung von Derivaten von Poly(2-propenal) und Poly(2-propensäure) mit Polyalkylenglykolen zur Behandlung und Prophylaxe von gastrointestinalen Erkrankungen bei Tieren.

Wu et al. offenbaren in Gastroenterology 2004, 126, Seiten 488-498, dass Polyethylenglykol mit einem Molekulargewicht von 15000 bis 20000 das intestinale Epithel bei Stress gegen die Invasion von *Pseudomonas aeruginosa* schützt.

Die Aufgabe der vorliegenden Erfindung besteht somit in der Bereitstellung eines weiteren Mittels, das an Vieh verfüttert werden kann, insbesondere zur Nahrungsergänzung, und Antibiotika ersetzen kann. Das Mittel soll dabei Infektionen verhindern, jedoch keine antibiotische Wirkung aufweisen und keine Resistenzen bei den mögliche Infektionen des Viehs verursachenden Bakterien hervorrufen.

Diese Aufgabe wird gelöst durch die Verwendung von Alkoxylaten ein- und mehrwertiger Alkohole oder eines Derivats davon als Antibiotikumersatz bei der Aufzucht und Haltung von Vieh.

Diese Aufgabe wird weiterhin gelöst durch die Verwendung von Alkoxylaten ein- und mehrwertiger Alkohole oder eines Derivats davon als antimikrobielle Substanz bei der Aufzucht und Haltung von Vieh.

Ebenfalls wird diese Aufgabe gelöst durch die Verwendung von Alkoxylaten ein- und mehrwertiger Alkohole oder eines Derivats davon als Futterergänzung bei der Aufzucht und Haltung von Vieh.

Bei den erfindungsgemäßen Verwendungen können die entsprechenden Alkoxylate ein- und mehrwertiger Alkohole oder Derivate davon mit dem Futter verabreicht werden (Futterergänzung), aber auch gegebenenfalls separat verfüttert werden. Die Futterergänzung ist der übliche und bevorzugte Weg, hier wird das Alkoxylat ein- und mehrwertiger Alkohole oder ein Derivat davon dem Futter beigemischt, das Vieh nimmt es während der Fütterung auf.

Durch das Verabreichen von Alkoxylaten ein- und mehrwertiger Alkohole oder eines Derivats davon wird eine hohe Gesundheit und eine verminderte Krankheitsanfälligkeit des Viehs erreicht. Dies ist sowohl ein ethischer als auch ein wirtschaftlicher Aspekt. Ebenfalls wird eine erhöhte Gewichtszunahme erreicht, was im Fall von Schlachtvieh einen wirtschaftlich ganz erheblichen Faktor darstellt.

"Erhöhte Gewichtszunahme" bezeichnet dabei das Phänomen, dass ein Tier in kürzeren Abständen die gleiche (oder eine höhere Menge) an Gewicht zulegt, als dies ohne den Zusatz der erfindungsgemäß verwendeten Alkoxylate ein- und mehrwertiger Alkohole oder eines Derivats davon, oder natürlich auch ohne den Zusatz von AMWF, der Fall ist. Die üblichen Werte der Gewichtszunahme sind natürlich stark von der Art des Viehs abhängig und dem Fachmann bekannt.

"Vieh" im Zusammenhang mit der vorliegenden Erfindung bezieht sich auf sämtliche Tiere, die von Menschen gehalten werden, ungeachtet des Zwecks. Die Haltung kann dabei eine Stall- oder Freilandhaltung sein, vorzugsweise eine Stallhaltung. Die vom Menschen gehaltenen Tiere sind dem Fachmann bekannt, Beispiele umfassen Schweine, Kühe, Geflügel, Schafe, Ziegen und Pferde.

Im Zusammenhang mit der vorliegenden Erfindung umfasst der Begriff "Kühe" Rinder, Ochsen, Kälber, Kühe und Milchkühe.

Im Zusammenhang mit der vorliegenden Erfindung umfasst der Begriff "Geflügel" Hühner, Hähne, Gänse, Puten und Enten.

Im Zusammenhang mit der vorliegenden Erfindung umfasst der Begriff "Schafe" Lämmer, Schafkühe und Schafbullen.

Bei dem Vieh kann es sich um Schlachtvieh, Nutzvieh (wie beispielsweise Milchkühe und Legehennen), oder auch sonstige, nicht zur Nahrungserzeugung, sondern zum Vergnügen oder für Wettkämpfe gehaltenes Vieh (beispielsweise Pferde) handeln.

Alkoxylat eines ein- oder mehrwertiger Alkohols oder eines Derivats davon wird im Zusammenhang mit der vorliegenden Erfindung gebraucht für eine Verbindung mit der Formel (1)

X(O-Y-Z)ₙ (I)

Dabei ist
- X =: H, C₁-C₂₄ -Alkyl, das gegebenenfalls einen oder mehrere OH-Substituenten und/oder Ketofunktionen und/oder Aldehydfunktionen beziehungsweise deren Derivate aufweist, C₅-C₇ -Cycloalkyl, Phenyl, Phenyl-(C₅-C₁₅-Alkyl)
- Y =: Polyalkylenglykol(co)polymereinheit aufgebaut aus identischen oder voneinander verschiedenen Monomereinheiten der Formel (R-O)
- Z =: H; C₁-C₂₄-Alkyl; -C(O)-R'
- R =: C₂-C₁₀-Alkyl
- R' =: C₁ - C₂₄-Alkyl oder C₂ - C₂₄-Alkenyl, wobei der Alkenylrest eine oder mehrere Doppelbindungen aufweisen kann
- n =: 1, 2, 3, 4, 5,6

"Alkyl" in Zusammenhang mit der vorliegenden Erfindung bedeutet lineare und verzweigte Alkylgruppen. Im Fall von X kann der C₁-C₂₄-Alkylrest auch ein von einem Keton oder Aldehyd abgeleiteter Rest sein.

"Alkenyl" in Zusammenhang mit der vorliegenden Erfindung bedeutet lineare und verzweigte Alkenylgruppen.

Wenn X ein C₂-C₂₄ -Alkylrest ist, der einen oder mehrere OH-Substituenten und eine Keto- oder Aldehydfunktion beziehungsweise deren Derivate aufweist, kann es sich um einen von Zuckern abgeleiteten Rest handeln, enthält der C₂-C₂₄-Rest statt der Keto- beziehungsweise Aldehydfunktion eine weitere OH-Gruppe, kann es sich um einen von Zuckeralkoholen abgeleiteten Rest handeln..

Vorzugsweise besitzen die Symbole folgende Bedeutung:
- X =: H, Methyl, Ethylen, Glyceryl, 2,2-Dimethylenpropylen
- R =: C₂-C₅-Alkyl, C₁₀-Alkyl
- Z =: H, C₁-C₅-Alkyl, C₁₀-Alkyl
- n =: 1,2,3.

Die Polyalkylenglykol(co)polymereinheit weist vorzugsweise ein Molekulargewicht von 1 500 - 10 000 auf.

Vorzugsweise weist die Polyalkylenglykol(co)polymereinheit Ethylenglykoleinheiten auf. Der Ethylenglykolgehalt der Polyalkylenglykol(co)polymereinheit liegt dabei vorzugsweise bei 80 - 100 Gew.-%.

Das oder die Alkoxylate ein- und/oder mehrwertiger Alkohole und/oder das Derivat oder die Derivate davon können einzeln oder als Gemisch von 2, 3 oder mehr Substanzen vorliegen.

Die Alkoxylate gemäß Formel (1) werden dem Futter in einer Konzentration von 0,1 - 5 Gew.-%, bevorzugt in einer Konzentration von 0,5 - 3 Gew.-% zugesetzt.

Das nach der vorliegenden Erfindung verabreichte Alkoxylat eines ein- oder mehrwertiger Alkohols oder eines Derivats davon oder die entsprechenden Gemische können gelöst oder in fester Form als Pulver, Schuppen oder Pellets, gegebenenfalls mit weiteren Zusatzstoffen vermischt, vorliegen.

Üblicherweise wird das Alkoxylat eines ein- oder mehrwertiger Alkohols oder dessen Derivats oder das entsprechende Gemisch mit dem Futter verabreicht. Vorzugsweise liegt das Futter in trockener Form vor. Das Alkoxylat eines ein- oder mehrwertiger Alkohols oder Derivats gemäß Formel (1) wird üblicherweise mit dem als Pulver oder Granulat vorliegenden Futter in Form von Pulver, Schuppen oder Pellets vermischt oder als Schmelze mit dem Futter vermischt und die Mischung extrudiert. Das Alkoxylat eines ein- oder mehrwertiger Alkohols oder das Derivat davon kann in Wasser gelöst oder darin suspendiert und/oder auf einem Träger angebracht sein. Das Alkoxylat kann durch Aufsprühen in flüssiger oder gelöster Form auf das Futter aufgebracht werden. Weitere Bestandteile des Futters, beispielsweise hydrophobe Substanzen wie Lipide oder Carbonsäuren, können vor oder nach der Zugabe der Alkoxylate ein- und mehrwertiger Alkohole oder deren Derivate davon dem Futter beigemengt werden. Weitere Möglichkeiten der Herstellung des Futters sind dem Fachmann bekannt.

Bei der Herstellung des Futters wird üblicherweise ein Vorgemisch hergestellt, das aus beispielsweise Vitaminen, Aromen, Mineralien und Enzymen besteht. Es ist möglich, einer solchen Formmischung trockene Komponenten zuzumischen, beispielsweise: gemahlener oder zerstoßener Weizen, Hafer, Gerste, Mais und Reis; pflanzliches Proteinfutter basierend auf beispielsweise Raps, Soja und Sonnenblume; tierisches Proteinfutter, beispielsweise Protein E, Blutmehl, Knochenmehl und Fischmehl, Molasse; Milchprodukte, beispielsweise verschiedene Milch- und Molkepulver. Zu diesem Vorgemisch wird üblicherweise das Alkoxylat eines ein- oder mehrwertiger Alkohols oder dessen Derivats gegeben, gegebenenfalls in Wasser gelöst oder emulgiert und/oder auf einem Träger angebracht, der beispielsweise aus gemahlenem Getreide, Stärke oder anorganischen Mineralien wie etwa Silikaten besteht. Nach Mischen der trockenen Additive kann die hydrophobe Verbindung in flüssiger Form, gegebenenfalls nach Erhitzen, zugefügt werden. Diese kann aus Lipiden bestehen, beispielsweise Fett oder Carbonsäuren, etwa Fettsäuren. Üblicherweise bestehen die Lipide aus Schlachtfett oder pflanzlichem Fett, gegebenenfalls nach Erhitzen. Nach sorgfältigem Vermischen wird eine Zusammensetzung in Form von Mehl oder in fester Form erhalten, abhängig vom Mahlgrad der Zutaten. Falls gewünscht, kann die in flüssiger Form vorliegende Komponente zu den festen, als Pulver oder Partikel vorliegenden Komponenten oder einem Teil davon gegeben werden, um eine feste pulverförmige Mischung zu erhalten, bevor das Alkoxylat ein- oder mehrwertiger Alkohole oder eines Derivats davon zugegeben wird.

Es ist ebenso möglich, das Futter in Form einer Suspension bereitzustellen. Dies ist insbesondere angebracht, wenn das Futter sofort verzehrt werden soll.

Das im Rahmen der vorliegenden Erfindung verabreichte Futter enthält zusätzlich zu dem Alkoxylat eines ein- und mehrwertiger Alkohols oder Derivats davon die folgenden Bestandteile: 0 bis 8 Gew.-%, vorzugsweise 10 bis 70 Gew.-% Getreide; 0 bis 30 Gew.-%, vorzugsweise 0 bis 15 Gew.-% und insbesondere 1 bis 8 Gew.-% Fett; 0 bis 85 Gew.-%, vorzugsweise 10 bis 50 Gew.-% proteinhaltiger, Nicht-Getreide-Nährsubstanzen; 0 bis 12 Gew.-%, vorzugsweise 1 bis 10 Gew.-% Vitamine, Mineralien, Enzyme und/oder Aromen; weiterhin gegebenenfalls andere übliche Bestandteile des jeweiligen Futters. Das Futter enthaltend das Alkoxylat eines ein- oder mehrwertiger Alkohols oder dessen Derivats wird vorzugsweise konfektioniert, beispielsweise durch Pelletieren, Expandieren oder Extrudieren. Gegebenenfalls noch vorhandenes Wasser kann danach durch Trocknen entfernt werden.

## Patentansprüche

1. Verwendung von Alkoxylaten ein- und mehrwertiger Alkohole und ihrer Derivate als Antibiotikaersatz bei der Aufzucht und Haltung von Vieh.

2. Verwendung nach Anspruch 1, **dadurch gekennzeichnet, dass** die Alkoxylate und ihre Derivate die allgemeine Formel (I)
X(O-Y-Z)ₙ (I)
mit
X = H, C₁-C₂₄ -Alkyl, das gegebenenfalls einen oder mehrere OH-Substituenten und/oder Ketofunktionen und/oder Aldehydfunktionen beziehungsweise deren Derivate aufweist, C₅-C₇ -Cycloalkyl, Phenyl, Phenyl-(C₅-C₁₅-Alkyl)
Y = Polyalkylenglykol(co)polymereinheit aufgebaut aus identischen oder voneinander verschiedenen Monomereinheiten der Formel (R-O)
Z = H; C₁-C₂₄-Alkyl; -C(O)-R'
R = C₂-C₁₀-Alkyl
R' = C₁ - C₂₄-Alkyl oder C₂ - C₂₄-Alkenyl, wobei der Alkenylrest eine oder mehrere Doppelbindungen aufweisen kann
n = 1, 2, 3, 4, 5,6
aufweisen.

3. Verwendung nach Anspruch 2, **dadurch gekennzeichnet, dass** in der allgemeinen Formel (1) die Symbole folgende Bedeutung haben:
X = H, Methyl, Ethylen, Glyceryl, 2,2-Dimethylenpropylen
R = C₂-C₅-Alkyl, C₁₀-Alkyl
Z = H, C₁-C₅-Alkyl, C₁₀-Alkyl
n = 1,2,3.

4. Verwendung nach Anspruch 2 oder 3, **dadurch gekennzeichnet, dass** die Polyalkylenglykolgruppen ein Molekulargewicht von 1 500 bis 10 000 aufweisen.

5. Verwendung nach einem der Ansprüche 2 bis 4, **dadurch gekennzeichnet, dass** der Ethylenglykolgehalt der Polyalkylenglykolgruppen bei 80 bis 100 Gew.-% liegt.

6. Verwendung nach einem der Ansprüche 1 bis 5, **dadurch gekennzeichnet, dass** es sich um Schlachtvieh, Nutzvieh oder sonstiges Vieh handelt.

7. Verwendung nach einem der Ansprüche 1 bis 6, **dadurch gekennzeichnet, dass** das Vieh ausgewählt ist aus Schweinen, Kühen, Geflügel, Schafen, Ziegen und Pferden.

8. Verwendung von Alkoxylaten ein- und mehrwertiger Alkohole und ihrer Derivate der allgemeinen Formel (1) als antimikrobielle Substanz bei der Aufzucht und Haltung von Vieh.

9. Verwendung von Alkoxylaten ein- und mehrwertiger Alkohole und ihrer Derivate der allgemeinen Formel (1) als Futterergänzung bei der Aufzucht von Vieh.

10. Verwendung nach einem der Ansprüche 1 bis 9, **dadurch gekennzeichnet, dass** eine rasche Gewichtszunahme erreicht wird.
